# EUROPEAN PATENT SPECIFICATION

(11) **EP 3 981 464 B1**
(45) Date of publication and mention of the grant of the patent: **18.11.2026**
(21) Application number: 21205684.0
(22) Date of filing: 16.05.2007
(51) Int. Cl.: A61N 1/39, A61N 1/05, A61N 1/36

(54) **SYSTEM TO REDUCE DEVICE THERAPY PAIN**
SYSTEM ZUR VERRINGERUNG VON BEHANDLUNGSSCHMERZEN
SYSTÈME POUR RÉDUIRE LA DOULEUR THÉRAPEUTIQUE D'UN DISPOSITIF

(30) Priority: 31.08.2006 US 469110
(43) Date of publication of application: 13.04.2022
(62) Document number(s) of the earlier application(s) in accordance with Art. 76 EPC: 07783809.2 / 2 056 929
(73) Proprietor: Cardiac Pacemakers, Inc., St. Paul, MN 55112-5798 (US)
(72) Inventor: ZHANG, Yunlong, Mounds View, MN, 55112 (US); SCHWARTZ, Mark, White Bear Lake, MN, 55110 (US)
(74) Representative: Pfenning, Meinig & Partner mbB

(56) References cited:
- US-A- 5 782 882
- US-A1- 2004 162 594
- US-A1- 2004 220 628
- US-A1- 2006 155 334
- US-B1- 6 438 418

## Description

### CLAIM OF PRIORITY

Benefit of priority is hereby claimed to U.S. Patent Application Serial Number 11/469,110, filed on August 31, 2006.

### FIELD

This application relates generally to medical devices and, more particularly, to systems, devices and methods for reducing device therapy pain.

### BACKGROUND

Medical devices can be implanted to deliver electrical therapy to portions of the body to help alleviate certain medical conditions. Some medical conditions that may benefit from electrical therapy may include atrial tachyarrhythmia and/or ventricular tachyarrhythmia. For example, implantable electrodes can be coupled to a pulse generator. The electrode can be implanted in or near the heart and electrical therapy such as cardioversion or defibrillation shocks can be delivered from the pulse generator to the heart via the electrode.

However, these shocks can cause physiological stress, anxiety, and pain to the patient. The anticipation of the shock can cause anxiety and distress. As well, the shock itself can be painful. There is a need to reduce the pain of the device therapy. Document US2004162594A1 describes a system for therapeutically activating tissue at a first site within a patient's body while blocking activation of tissue or conduction of action potentials at a second site to reduce undesired side effects. The system uses high frequency stimulation and/or direct current pulses to block activation at the second site. Various applications include promoting micturition, defecation, peristalsis, and defibrillation while preventing associated undesired effects such as sphincter contraction and pain.

### SUMMARY

The invention is defined by the appended claims.

One aspect includes sensing whether a shock to a heart is needed, and prior to delivering the shock, causing at least a partial contraction of a musculature in a chest, and delivering the shock while the musculature is at least partially contracted.

One aspect includes sensing whether a therapeutic shock to a heart is needed, and before delivering the therapeutic shock, applying subcutaneous electrical neural stimulation near a pectoris major muscle.

### BRIEF DESCRIPTION OF THE DRAWINGS

Figure 1 shows a system to reduce device therapy pain, in accordance with certain embodiments.
Figure 2 shows a system to reduce device therapy pain, in accordance with certain embodiments.
Figure 3 shows a method to reduce device therapy pain, in accordance with certain embodiments.
Figure 4 shows a system to reduce device therapy pain, in accordance with certain embodiments.
Figure 5 shows a system to reduce device therapy pain, in accordance with certain embodiments.
Figure 6 shows a method to reduce device therapy pain, in accordance with certain embodiments.

### DETAILED DESCRIPTION

The following detailed description and accompanying drawings show specific embodiments in which the present invention may be practiced. These embodiments are described in sufficient detail to enable those skilled in the art to practice the invention. Aspects of these embodiments can be combined and other embodiments may be utilized and structural changes may be made without departing from the scope of the present invention.

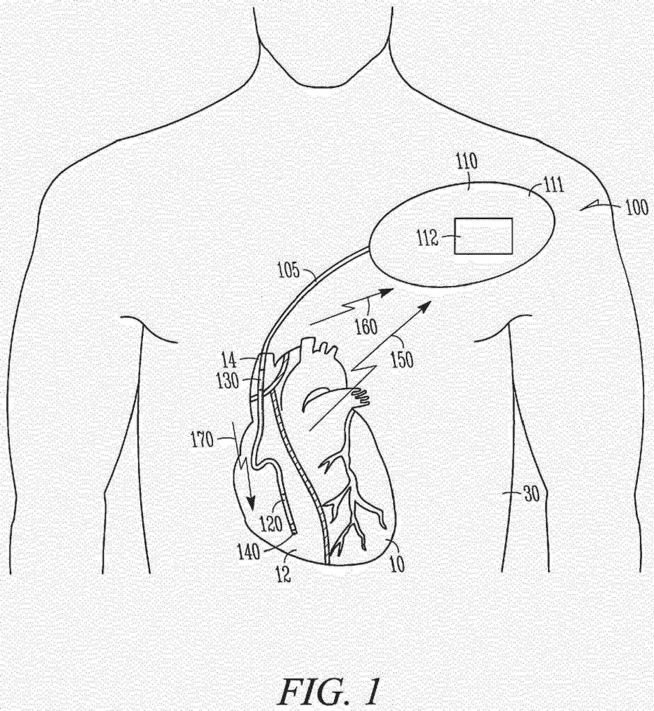

Figure 1 shows a system 100 for reducing pain during device therapy. System 100 generally includes a pulse generator 110 with at least one electrode 120 operatively coupled to the pulse generator. Electrode 120 is a defibrillation or cardioversion electrode to deliver defibrillation pulses to a heart 10. The electrode can be located in the ventricle 12 or the atrium 14. Other embodiments can include a second defibrillation electrode 130, and/or a third electrode 140, such as a sensing electrode. More than three electrodes can be used. Electrodes 120, 130, and 140 can be disposed along a lead 105 which is coupled to pulse generator 110.

Pulse generator 110 includes controller circuitry 112 to control the functions of the pulse generator. Controller circuitry 112 can include hardware, software, and/or combinations of hardware and software programmed to deliver therapy shocks to heart 10, via one or more of electrodes 120, 130, and 140, such as atrial or ventricle defibrillation shocks or cardioversion shocks. In certain embodiments, pulse generator housing 111 can act as an electrode also.

According to the invention, pulse generator 110 is further adapted to deliver one or more pre-therapy conditioning electrical pulse or pulses to stimulate the chest musculature to reduce the startle effect from the muscle contraction from the shock in turn reducing the pain and/or anxiety caused by the therapy shock discussed above.

According to the invention, pulse generator 110 delivers one or more pre-therapy conditioning electrical pulses which are directed so as to cause a preconditioning of the chest musculature similar to a fused tetanus condition of a musculature in a chest 30. A fused tetanus condition is when the chest musculature is at least partially contracted. Accordingly, the one or more pre-therapy conditioning electrical pulses cause at least a partial contraction of the musculature of the chest. The one or more pre-therapy conditioning electrical pulse or pulses are applied in such a manner that the chest musculature is still in the at least partially contracted state when the therapy shock is delivered. According to the invention, the one or more pre-therapy conditioning electrical pulse or pulses are applied recurrently or continually at least until the shock therapy is delivered. In certain embodiments, the pre-therapy conditioning electrical pulse or pulses can be applied recurrently or continually from a time prior to the therapy shock and continue so that the pulses partially or completely overlap with the therapy shock. This chest muscle contraction results in lessened pain once the therapy shock is delivered since the chest is pre-contracted. Thus, the patient is less surprised by the therapy shock and the muscles are not contracted as much by the therapy shock itself.

The one or more pre-therapy conditioning electrical pulses can be directed in different fashions. In certain embodiments, pre-therapy conditioning electrical pulses 150 are directed directly at the musculature of the chest from electrode 120 to pulse generator housing 111. In certain embodiments, pre-therapy conditioning electrical pulses 160 are directed at the musculature of the chest from electrode 130 to pulse generator housing 111. In certain embodiments, pre-therapy conditioning electrical pulses 170 are directed at the musculature of the chest from electrode 130 to electrode 120. The electrical pulses can be directed at musculature on the right side of the chest, the left side of the chest, or both sides.

In some embodiments, separate pulse generators are used to deliver therapy shocks and to apply pre-therapy conditioning pulses.

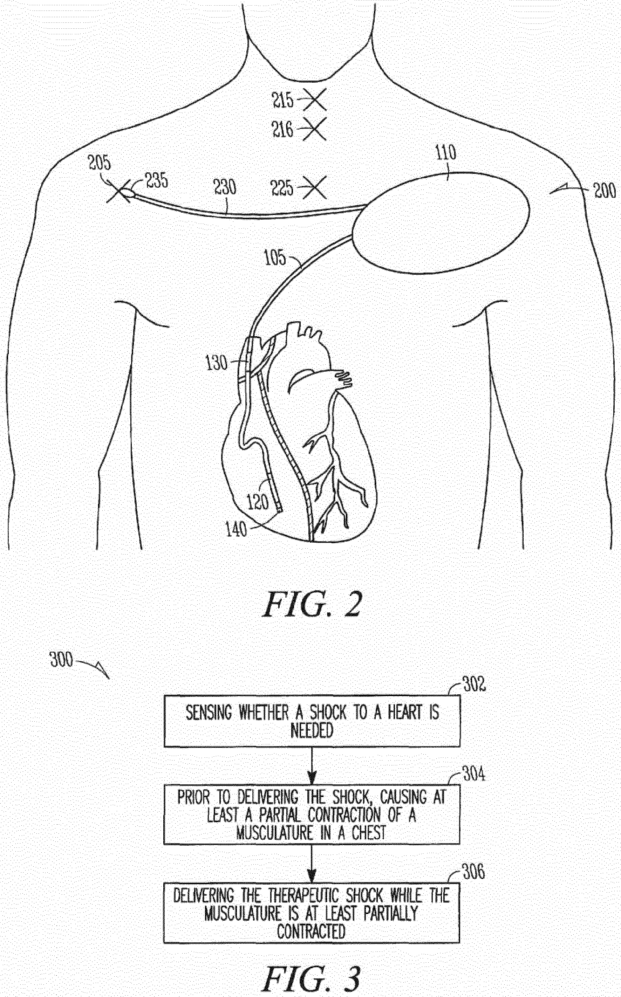

Figure 2 shows a system 200 to reduce device therapy pain, in accordance with certain embodiments. In this example, pulse generator 110 and electrode 120 can be implanted as discussed above. In this embodiment, an electrode 235 disposed along a second lead 230 is used to apply one or more pre-therapy conditioning electrical pulses. In this example, the pre-therapy conditioning electrical pulse or pulses are directed at one or more somatic nerves, such as anterior thoracic nerve 205. When a somatic nerve is signaled, the somatic nerve can cause the fused tetanus condition or chest muscle contraction, as discussed above. The somatic nerves control contraction of the chest muscles and by stimulating them at least up until the therapy shock is delivered, the patient's chest muscles are at least partially contracted and this lessens surprise and lessens the pain of contraction caused by the shock therapy itself.

Electrode 235 can be positioned to deliver therapy to a somatic nerve located anywhere in the body. In various embodiments, for example, electrode 235 can be implanted near or directed to deliver therapy to one or more somatic nerves in the back of the neck (the C7 upper subscapular nerve 215, or the C8 nerve 216, for example), the first thoracic spinal nerve T1 225 or the anterior thoracic nerve 205. Other embodiments can target other somatic nerve locations. Electrode 235 can include a tip electrode, a ring electrode, or a nerve cuff electrode, for example. In certain embodiments, an epidural nerve root stimulation lead can be used.

In some embodiments, separate pulse generators or electrodes are used to deliver therapy shocks and to apply pre-therapy conditioning pulses.

Figure 3 shows a method 300, in accordance with certain embodiments. Method 300 includes sensing whether a shock is needed (302), prior to delivering the shock, causing at least a partial contraction of a musculature in a chest (304); and delivering the therapeutic shock while the musculature is at least partially contracted (306).

In one example, as discussed above in Figure 1, the pre-therapy conditioning pulses are directed at the musculature of the chest, and are at a level so as to cause at least a partially contracted condition in the chest muscles. In certain embodiments, as discussed above in Figure 2, the pre-therapy conditioning electrical pulses include electrical pulses directed at somatic nerves such that the somatic nerves cause the fused tetanus condition. Some embodiments combine these two techniques. The somatic nerves can be stimulated in the back of the neck (the C7 upper subscapular nerve, or the C8 nerve, for example), the first thoracic spinal nerve (T1) or an anterior thoracic nerve.

The pre-therapy conditioning pulses directed at either the chest muscular, the somatic nerves, or both, are designed to at least partially contract the chest musculature in a fused tetanus condition. As discussed above, this precontraction of the chest muscles prepares the muscles for the therapy shock and helps reduce the startle reaction and/or the painful muscle contraction a patient feels when the therapy shock is delivered. The pre-therapy conditioning pulse or pulses are delivered at such a level and/or time that the chest muscles are still contracted when the therapy shock is delivered. Since the chest muscles are contracted right up until the shock therapy is delivered, the impact of the therapy shock on the musculature is reduced. In some examples, there can be up to about a 25 ms gap of time between the last of the one or more pre-therapy pulses and the beginning of the therapeutic shock since the muscle fibers will remain contracted.

In certain embodiments, sensing whether a shock is needed (302) includes sensing one or more electrical signals of a heart to determine if therapy is needed. For example, the pulse generator can include programming to recognize the need for atrial defibrillation or ventricle defibrillation, or any other shock therapy. When the pulse generator recognizes such a need, it then begins preparing to deliver the shock by charging a capacitor to deliver the shock therapy, which is typically between 1 J and 25 Joules, for example. The shock therapy can include cardioversion or defibrillation shocks for example.

In certain embodiments, the system can sense whether the therapy shock was successful. If the therapy was unsuccessful, then the system can repeat the procedure and provide the one or more pre-therapy condition pulses as many times as necessary.

In one embodiment, the pre-conditioning pulses of either embodiment discussed above can be in the about 20 to about 30 millisecond range, while the energy of the pulse will depend on the type of electrodes used. It is desired that the lowest energy be used to cause contraction. This can vary by person and electrodes used, since the voltage perception varies between patients (5V - 50V, for example), and the pain perception varies as well (50V - 200V, for example). Accordingly, in one embodiment, the device is programmable to deliver between about 5V to about 200V. According to the invention, the pre-therapy conditioning is programmed anywhere from about 10 Hz to about 100 Hz.

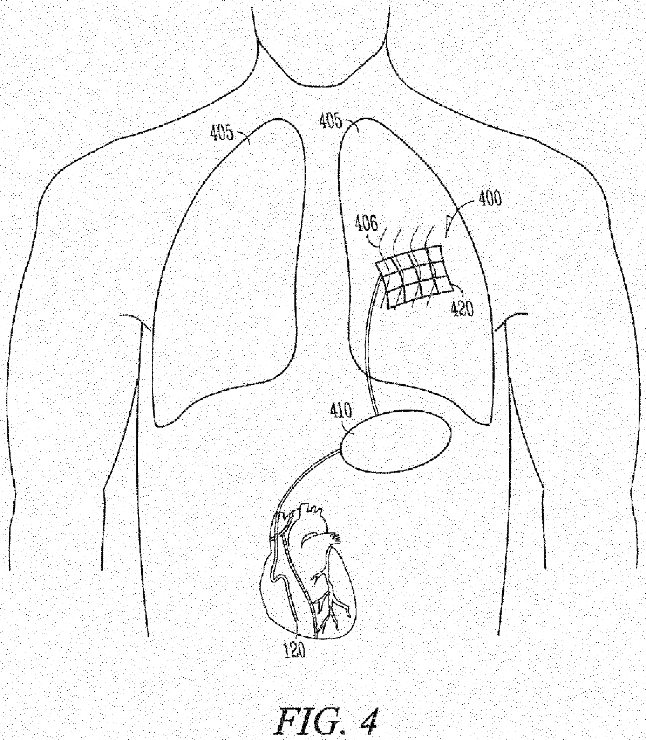

Figure 4 shows a system 400 to reduce device therapy pain, in accordance with certain embodiments. System 400 includes a pulse generator 410 and an electric mesh 420 adapted to deliver subcutaneous electrical neural stimulation prior to delivery of the shock therapy. An electrode 120 can further be couple to the pulse generator 410 to deliver the therapy shocks. In some embodiments, separate pulse generators or electrodes are used to deliver therapy shocks and to apply the subcutaneous electrical neural stimulation.

In certain embodiments, system 400 includes electrical mesh 420 implanted near or on the pectoris major muscles 405. In certain embodiments, applying subcutaneous electrical neural stimulation includes applying electrical pulses which stimulate pain nerve fibers 406 in the pectoris major muscles 405. In other embodiments, mesh 420 can be on the other side of the chest, or there can be an electrical mesh on both sides of the chest. By stimulating these nerve fibers, system 400 provides sedative therapy prior to and/or during and after the therapy shock. For example, the system can provide a pain signal conduction block, or can modulate pain signal transmission, and/or stimulate pain nerve fibers at a level so as to induce endogenous analgesics. This masks the pain and results in less pain from the subsequent therapy shock. This approach can help reduce pain, spasm and/or tetanus in the chest muscles.

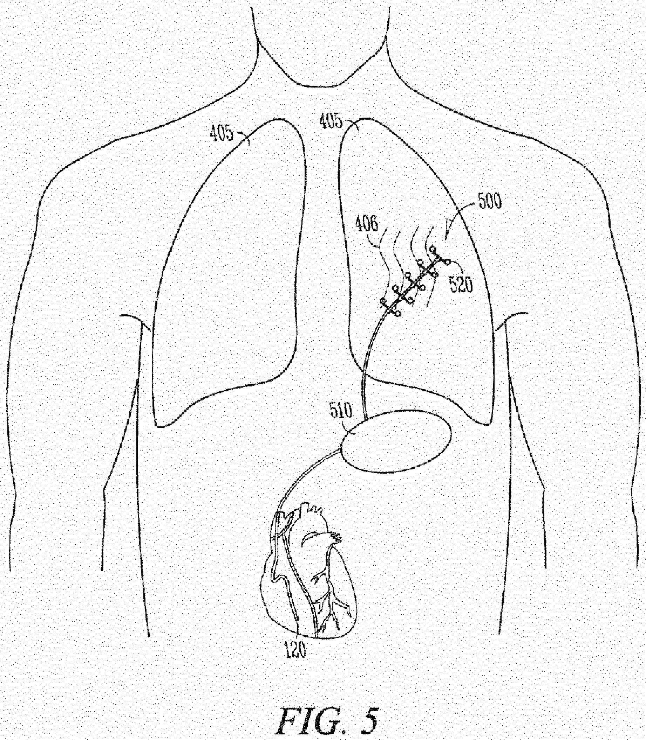

Figure 5 shows a system 500 to reduce device therapy pain, in accordance with certain embodiments. System 500 includes a pulse generator 510 and an electrode array 520 adapted to deliver subcutaneous electrical neural stimulation prior to delivery of the shock therapy. An electrode 120 can further be couple to the pulse generator 510 to deliver the therapy shocks, such as discussed above. In some embodiments, separate pulse generators or electrodes are used to deliver therapy shocks and to apply subcutaneous electrical neural stimulation.

In certain embodiments, system 500 includes electrode array 520 implanted near or on the pectoris major muscles 405. In certain embodiments, applying subcutaneous electrical neural stimulation includes applying electrical pulses which stimulate pain nerve fibers 406 in the pectoris major muscles 405. By stimulating these nerve fibers, the system provides a pain signal conduction block, modulates pain signal transmission, and/or stimulates pain nerve fibers at a level so as to induce endogenous analgesics. This results in less pain from the subsequent therapy shock.

Referring to both Figures 4 and 5, applying subcutaneous electrical neural stimulation can include the following stimulus parameters: an amplitude current at low intensity just above the threshold. A pulse width (duration) of about 10-1000 microseconds. A pulse rate (frequency) of about 40-150 impulses per second (Hz). One embodiment uses a frequency of about 80-100 Hz; or a pulse rate of about 0.5 - 10 Hz if the stimulus amplitude current is set at a high intensity. One embodiment uses a high stimulation frequency (about 40-150 Hz) and a low intensity, just above threshold, with the current set between about 10 - 30 mA. The pulse duration can be short (up to about 50 microseconds, for example). The onset of analgesia with this setup is almost immediate. Pain relief lasts while the stimulus is tuned on, and usually abates after the stimulation stops.

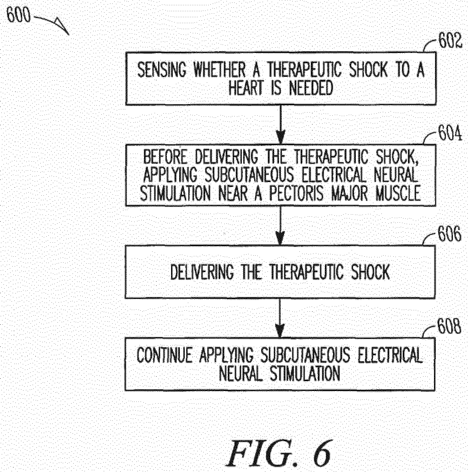

Figure 6 shows a method 600 to reduce therapy pain. Method 600 includes sensing whether a therapeutic shock is needed (602), before delivering the therapeutic shock, applying subcutaneous electrical neural stimulation (604), delivering the therapeutic shock (606), and continuing to deliver the subcutaneous electrical neural stimulation after the therapeutic shock (608). Certain embodiments omit continuing to deliver the subcutaneous electrical neural stimulation after the therapeutic shock.

As discussed above, sensing whether a therapeutic shock is needed includes sensing electrical signals of a heart. For example, the pulse generator can include programming to recognize the need for atrial or ventricle defibrillation. When the pulse generator recognizes such a need, it then begins preparing to deliver the shock by charging a capacitor to deliver the shock therapy, which is typically between 1 J and 25 Joules, for example. The shock therapy can include cardioversion or defibrillation shocks for example.

As discussed above, subcutaneous electrical neural stimulation can be applied via an electrical mesh implanted near the pectoris major muscles, or by an electrode array, for example. In certain embodiments, applying subcutaneous electrical neural stimulation includes applying electrical pulses which stimulate pain nerve fibers in the pectoris major muscles. By stimulating these nerve fibers, the method provides a pain signal conduction block, or modulates pain signal transmission, and/or stimulates pain nerve fibers at a level so as to induce endogenous analgesics.

In other embodiments, the pulse generator controller circuitry discussed above can be within the pulse generator or be a separate system communicating wirelessly with the pulse generator. In some embodiments, the pulse generator can include a transceiver and associated circuitry for use to communicate with a programmer or another external or internal device. Various embodiments can include wireless communication capabilities. For example, some transceiver embodiments use a telemetry coil to wirelessly communicate with a programmer or another external or internal device. Certain embodiments can utilize far-field ICDs.

The above description is intended to be illustrative, and not restrictive. Many other embodiments will be apparent to those of skill in the art upon reviewing the above description. The scope of the invention should, therefore, be determined with reference to the appended claims.

## Claims

1. A system (100) to reduce device therapy pain, comprising:
a pulse generator (110); and
at least one electrode (120, 130, 140) operatively coupled to the pulse generator (110), wherein, in response to a need for shock therapy including a defibrillation shock or a cardioversion shock, the pulse generator (110) is adapted to deliver a plurality of pre-therapy conditioning electrical pulses via the electrode (120, 130, 140) to a chest musculature innervated by somatic nerves or to one or more somatic nerves innervating the chest musculature so as to cause at least a partial contraction in the chest musculature in a fused tetanus condition, and wherein the pulse generator (110) is configured to apply the plurality of pre-therapy conditioning electrical pulses recurrently at a frequency of between 10 Hz to 100 Hz from a time prior to delivery of the shock therapy at least until the shock therapy including a defibrillation shock or a cardioversion shock is delivered.

2. The system (100) of claim 1, wherein the pulse generator (110) also delivers the shock therapy.

3. The system (100) of any one of claim 1 or 2, wherein further comprising means for sensing one or more electrical signals of the heart to determine a need for shock therapy.

4. The system (100) of any one of the preceding claims, wherein the pre-therapy conditioning electrical pulses from the pulse generator (110) include pulses between about 5V to about 200V.

5. The system (100) of any one of the preceding claims, wherein the pre-therapy conditioning electrical pulses from the pulse generator (110) include pulses between about 20 milliseconds to about 30 milliseconds.

6. The system (100) of any one of claims 1-3, wherein the pre-therapy conditioning electrical pulses are between about 5V to about 200V and between about 20 milliseconds to about 30 milliseconds.

7. The system (100) of any one of the preceding claims, wherein there is a gap of time between the last of the one or more pre-therapy pulses and the beginning of the therapeutic shock of up to about 25 ms.

8. The system (100) of any one of the preceding claims, wherein the at least one electrode (120, 130, 140) comprises at least one defibrillation or cardioversion electrode (120, 130, 140) disposed along a first lead (105) connected to the pulse generator (110), wherein the at least one defibrillation or cardioversion electrode (120, 130, 140) is configured to deliver a defibrillation shock or a cardioversion shock to a heart.

9. The system (100) of claim 8, wherein the system (100) comprises an electrode (235) disposed along a second lead (230) and configured to deliver the pre-therapy conditioning electrical pulses to the one or more somatic nerves.

10. The system (100) of claim 9, wherein the second lead (230) includes an epidural nerve root stimulation lead.

11. The system (100) of any one of claims 9 or 10, wherein the electrode (235) includes a tip electrode, a ring electrode or a nerve cuff.

## Patentansprüche

1. System (100) zur Reduzierung von Therapieschmerzen, umfassend:
einen Pulsgenerator (110); und
mindestens eine Elektrode (120, 130, 140), die operativ mit dem Pulsgenerator (110) gekoppelt ist, wobei der Pulsgenerator (110) angepasst ist, in Reaktion auf einen Bedarf an einer Schocktherapie, einschließlich eines Defibrillationsschocks oder eines Kardioversionsschocks, eine Vielzahl von pre-therapeutischen elektrischen Konditionierungspulsen über die Elektrode (120, 130, 140) an eine durch somatische Nerven innervierte Brustmuskulatur oder an einen oder mehrere somatische Nerven, die die Brustmuskulatur innervieren, abzugeben, um zumindest eine teilweise Kontraktion in der Brustmuskulatur in einem fusionierten Tetanus-Zustand zu bewirken, und wobei der Pulsgenerator (110) eingerichtet ist, die mehreren pre-therapeutischen elektrischen Konditionierungspulse wiederholt mit einer Frequenz zwischen 10 Hz und 100 Hz ab einem Zeitpunkt vor der Abgabe der Schocktherapie mindestens bis zur Abgabe der Schocktherapie, einschließlich eines Defibrillationsschocks oder eines Kardioversionsschocks, abzugeben.

2. System (100) nach Anspruch 1, wobei der Pulsgenerator (110) auch die Schocktherapie abgibt.

3. System (100) nach einem der Ansprüche 1 oder 2, wobei es ferner Mittel zum Erfassen eines oder mehrerer elektrischer Signale des Herzens umfasst, um die Notwendigkeit einer Schocktherapie zu bestimmen.

4. System (100) nach einem der vorangehenden Ansprüche, wobei die pre-therapeutischen Konditionierungspulse von dem Pulsgenerator (110) Pulse zwischen etwa 5 V bis etwa 200 V einschließen.

5. System (100) nach einem der vorangehenden Ansprüche, wobei die pre-therapeutischen Konditionierungspulse aus dem Pulsgenerator (110) Pulse zwischen etwa 20 Millisekunden bis etwa 30 Millisekunden einschließen.

6. System (100) nach einem der Ansprüche 1 bis 3, wobei die pre-therapeutischen Konditionierungspulse eine Spannung zwischen etwa 5 V und etwa 200 V sowie eine Dauer zwischen etwa 20 Millisekunden und etwa 30 Millisekunden aufweisen.

7. System (100) nach einem der vorstehenden Ansprüche, wobei zwischen dem letzten der einen oder mehreren pre-therapeutischen Pulse und dem Beginn des therapeutischen Schocks eine Zeitlücke von bis zu etwa 25 ms besteht.

8. System (100) nach einem der vorstehenden Ansprüche, wobei die mindestens eine Elektrode (120, 130, 140) mindestens eine Defibrillations- oder Kardioversionselektrode (120, 130, 140) umfasst, die entlang einer ersten Leitung (105) angeordnet ist, die mit dem Pulsgenerator (110) verbunden ist, wobei die mindestens eine Defibrillations- oder Kardioversionselektrode (120, 130, 140) eingerichtet ist, einen Defibrillations- oder Kardioversionsschock an ein Herz abzugeben.

9. System (100) nach Anspruch 8, wobei das System (100) eine Elektrode (235) umfasst, die entlang einer zweiten Leitung (230) angeordnet und eingerichtet ist, die elektrischen Pulse zur Konditionierung vor der Therapie an den einen oder die mehreren somatischen Nerven abzugeben.

10. System (100) nach Anspruch 9, wobei die zweite Elektrode (230) eine Elektrode zur epiduralen Nervenwurzelstimulation umfasst.

11. System (100) nach einem der Ansprüche 9 oder 10, wobei die Elektrode (235) eine Spitzenelektrode, eine Ringelektrode oder eine Nervenmanschette umfasst.

## Revendications

1. Système (100) destiné à réduire la douleur associée à une thérapie par dispositif, comprenant :
un générateur d'impulsions (110) ; et
au moins une électrode (120, 130, 140) qui est couplée de manière opérationnelle au générateur d'impulsions (110), dans lequel, en réponse à un besoin d'une thérapie par choc qui inclut un choc de défibrillation ou un choc de cardioversion, le générateur d'impulsions (110) est adapté pour délivrer, par l'intermédiaire de l'électrode (120, 130, 140), une pluralité d'impulsions électriques de conditionnement de pré-thérapie à une musculature thoracique qui est innervée par des nerfs somatiques ou à un ou plusieurs nerfs somatiques qui innervent la musculature thoracique de manière à provoquer au moins une contraction partielle de la musculature thoracique dans un état de tétanos fusionné ; et dans lequel le générateur d'impulsions (110) est configuré pour appliquer la pluralité d'impulsions électriques de conditionnement de pré-thérapie de manière récurrente à une fréquence comprise entre 10 Hz et 100 Hz à partir d'un instant qui est antérieur à la délivrance de la thérapie par choc et au moins jusqu'à ce que la thérapie par choc qui inclut un choc de défibrillation ou un choc de cardioversion soit délivrée.

2. Système (100) selon la revendication 1, dans lequel le générateur d'impulsions (110) délivre également la thérapie par choc.

3. Système (100) selon l'une quelconque des revendications 1 ou 2, comprenant en outre des moyens pour détecter un signal électrique ou plusieurs signaux électriques du cœur afin de déterminer un besoin d'une thérapie par choc.

4. Système (100) selon l'une quelconque des revendications précédentes, dans lequel les impulsions électriques de conditionnement de pré-thérapie en provenance du générateur d'impulsions (110) incluent des impulsions comprises entre environ 5 V et environ 200 V.

5. Système (100) selon l'une quelconque des revendications précédentes, dans lequel les impulsions électriques de conditionnement de pré-thérapie en provenance du générateur d'impulsions (110) incluent des impulsions délivrées pendant une durée comprise entre environ 20 millisecondes et environ 30 millisecondes.

6. Système (100) selon l'une quelconque des revendications 1 à 3, dans lequel les impulsions électriques de conditionnement de pré-thérapie sont comprises entre environ 5 V et environ 200 V et sont délivrées pendant une durée comprise entre environ 20 millisecondes et environ 30 millisecondes.

7. Système (100) selon l'une quelconque des revendications précédentes, dans lequel il y a un intervalle de temps qui peut aller jusqu'à environ 25 ms entre la dernière des une ou plusieurs impulsions électriques de conditionnement de pré-thérapie et le début du choc thérapeutique.

8. Système (100) selon l'une quelconque des revendications précédentes, dans lequel l'au moins une électrode (120, 130, 140) comprend au moins une électrode de défibrillation ou de cardioversion (120, 130, 140) qui est disposée le long d'une première sonde (105) qui est connectée au générateur d'impulsions (110) ; et dans lequel l'au moins une électrode de défibrillation ou de cardioversion (120, 130, 140) est configurée pour délivrer un choc de défibrillation ou un choc de cardioversion au cœur.

9. Système (100) selon la revendication 8, dans lequel le système (100) comprend une électrode (235) qui est disposée le long d'une seconde sonde (230) et qui est configurée pour délivrer les impulsions électriques de conditionnement de pré-thérapie aux un ou plusieurs nerfs somatiques.

10. Système (100) selon la revendication 9, dans lequel la seconde sonde (230) inclut une sonde de stimulation épidurale des racines nerveuses.

11. Système (100) selon l'une quelconque des revendications 9 ou 10, dans lequel l'électrode (235) inclut une électrode d'extrémité, une électrode annulaire ou une électrode en manchon pour nerf(s).
